(19) Europäisches Patentamt
European Patent Office
Office européen des brevets

(11) Veröffentlichungsnummer: **0 150 280**
**B1**

(12) **EUROPÄISCHE PATENTSCHRIFT**

(45) Veröffentlichungstag der Patentschrift: **02.05.90**

(51) Int. Cl.$^5$: **C 07 C 41/50**

(21) Anmeldenummer: **84113390.3**

(22) Anmeldetag: **07.11.84**

(54) **Verfahren zur Herstellung von Acetalen.**

(30) Priorität: **01.02.84 DE 3403426**

(43) Veröffentlichungstag der Anmeldung:
**07.08.85 Patentblatt 85/32**

(45) Bekanntmachung des Hinweises auf die Patenterteilung:
**02.05.90 Patentblatt 90/18**

(84) Benannte Vertragsstaaten:
**AT BE CH DE FR GB IT LI NL SE**

(56) Entgegenhaltungen:
**EP-A-0 079 432**
**FR-A-1 174 085**
**GB-A- 970 371**
**JP-A-5 790 873**
**US-A-3 641 163**

(73) Patentinhaber: **Degussa Aktiengesellschaft**
**Weissfrauenstrasse 9**
**D-6000 Frankfurt am Main 1 (DE)**

(72) Erfinder: **Andrade, Juan, Dr. Dipl.-Chem.**
**Hungerweg 5**
**D-8752 Kleinostheim (DE)**
Erfinder: **Arntz, Dietrich, Dr. Dipl.-Chem.**
**Lorsbachstrasse 32**
**D-6370 Oberursel (DE)**
Erfinder: **Kraft, Michael**
**Heinrich Heine Strasse 7**
**D-6458 Rodenbach 1 (DE)**
Erfinder: **Prescher, Günter, Dr. Dipl.-Chem.**
**Liesingstrasse 2**
**D-6450 Hanau 9 (DE)**

**EP 0 150 280 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zur Herstellung von Acetalen durch Umsetzung von Aldehyden mit Alkoholen in flüssiger Phase in Gegenwart fester saurer Katalysatoren.

Es ist bekannt, das Diäthylacetal des Acroleins herzustellen, indem das Acrolein mit dem Äthylester der Orthoameisensäure umgesetzt wird (Org. Synth. Coll. Vol. 4 (1963), 21). Das Verfahren ergibt zwar günstige Ausbeuten, ist aber sehr aufwendig und daher für eine Anwendung in technischem Maßstab ungeeignet.

Es ist auch bekannt, daß bei der Einwirkung von Alkoholen auf Aldehyde in Gegenwart von Katalysatoren Acetale gebildet werden. Insbesondere erfolgt diese Umsetzung im Falle des Acroleins in Gegenwart von starken Säuren, wie Schwefelsäure, p-Toluolsulfonsäure oder Trichloressigsäure, in mit Wasser nicht mischbaren organischen Lösungsmitteln, wie aliphatischen oder aromatischen, gegebenenfalls halogenierten, Kohlenwasserstoffen unter laufender Abführung des sich bildenden Wassers als azeotrope Mischung mit dem Lösungsmittel (DE—PS—930 752, US—PS 2691049). Nachteilig ist bei diesen Verfahren, daß Umsetzungszeiten von mehr als 10 Stunden benötigt werden. Zudem verläuft die Umsetzung wenig selektiv; es werden in erheblichem Umfang Nebenprodukte gebildet und die Ausbeuten sind, insbesondere im Falle der Herstellung des Dimethylacetals des Acroleins, unbefriedigend.

Es ist außerdem bekannt, im Falle der Umsetzung gesättigter oder ungesättigter aliphatischer Aldehyde mit zweiwertigen aliphatischen Alkoholen saure Ionenaustauscherharze als Katalysatoren zu verwenden (US—PS 4024159). Saure Ionenaustauscherharze dienen ebenfalls für die Umsetzung gesättigter aliphatischer Aldehyde mit einwertigen Alkoholen (JP—OS 57908/73). Bei diesem Verfahren verläuft die Umsetzung selektiver und es können Umsätze erzielt werden, die den nach den Umsetzungsgleichgewichten erzielbaren Umsätzen nahekommen. Da jedoch vorgesehen ist, die Gewinnung der Acetale in herkömmlicher Weise, inbesondere durch Destillation aus den Umsetzungsgemischen, vorzunehmen und hierbei Nebenreaktionen eintreten, können nur unbefriedigende Ausbeuten erwartet werden. Zudem wird über die Rückgewinnung und Wiederverwendung der nicht umgesetzten Anteile der Ausgangssubstanzen nichts ausgesagt. Auf die Rückgewinnung zu verzichten, bedeutet, daß das Verfahren schon deswegen unwirtschaftlich und für eine Anwendung in technischem Maßstab ungeeignet ist.

Aus der GB—A—970 371 ist ein Verfahren bekannt, bei dem man Alkohole mit Alkenalen in Gegenwart von mit Wasser nicht mischbaren organischen Lösungsmitteln bei temperaturen $<25°C$ umsetzt.

Das entstehende Acetal geht sofort in diese organische Phase über und wird somit aus dem Reaktionsgleichgewicht entfernt.

Nach dem Ende der Umsetzung trennt man die wässrige Phase ab und wäscht die organische mit Wasser, um restlichen Alkohol aus ihr zu entfernen.

Die EP—A—0 079 432 betrifft u.a. ein Verfahren zur Acetalisierung von Isobutyraldehyd mit Methanol, die entweder bei 60°C in Gegenwart von p-Toluolsulfonsäure oder bei 100°C und 5 der Betriebsdruck mit einem sauren Ionenaustauscher als Katalysator abläuft.

Man erhält ein aus einer wässrigen und einer organischen Phase bestehendes Reaktionsgemisch.

Die organische Phase enthält das Reaktionsprodukt und wird mehrmals mit Wasser gewaschen, um nicht umgesetztes Methanol und Isobutyraldehyd abzutrennen.

Aufgabe der Erfindung ist, ein Verfahren zur Herstellung von Acetalen bereitzustellen, bei dem man wasserhaltige Umsetzungsgemische, die frei von mit Wasser nicht mischbaren organischen Lösungsmitteln sind, kontinuierlich so aufarbeitet, daß sowohl bessere Umsätze als auch höhere Selektivitäten in Bezug auf des Acetal resultieren.

Gegenstand der Erfindung ist ein Verfahren zur Herstellung von Acetaten durch Umsetzung von Aldehyden der Formel

$$R_1—CHO \quad (I),$$

in der $R_1$ für den Methyl- oder Ethylrest steht oder eine Alkenylgruppe mit 2 oder 3 Kohlenstoffatomen bedeutet, mit

Alkoholen der Formel

$$R_2—OH \quad (II),$$

in der $R_2$ eine Alkylgruppe mit 1 oder 2 Kohlenstoffatomen bedeutet, in Gegenwart fester saurer Katalysatoren, die nach der Umsetzung abgetrennt werden, in flüssiger Phase in Abwesenheit zusätzlicher organischer Lösungsmittel, das dadurch gekennzeichnet ist,

a) daß das von dem Katalysator abgetrennte Umsetzungsgemisch kontinuierlich im Gegenstrom extrahiert wird, wobei man je Volumenteil des Umsetzungsgemisches 1 bis 4 Volumenteile Wasser und 1 bis 3 Volumenteile mit Wasser nicht mischbarer, organischer Lösungsmittel einsetzt, und

b) aus den Extrakten die gebildeten Acetale sowie die nicht umgesetzten Anteile der Aldehyde und Alkohole gewinnt.

Dieses Verfahren ergibt nicht nur günstige Umsätze, sondern ist auch hervorragend selektiv. Die gebildeten Acetale sowie die nicht umgesetzten Anteile der Aldehyde und Alkohole werden auf einfache Weise aus den Umsetzungsgemischen abgetrennt und mit sehr guten Ausbeuten gewonnen.

Zur Umsetzung nach dem erfindungsgemäßen Verfahren geeignete Aldehyde sind beispielsweise Propionaldehyd, insbesondere Acetaldehyd, Acrolein und Methacrolein, Geeignete Alkohole sind Äthanol und insbesondere Methanol.

Das erfindungsgemäße Verfahren dient demgemäß zur Herstellung von Acetalen der Formel

$$R_1—CH—(OR_2)_2 \qquad III$$

in der $R_1$ und $R_2$ die zu den Formeln I und II angegebenen Bedeutungen haben. Besonders geeignet ist das Verfahren für die Herstellung der Dimethylacetate des Acetaldehyds, des Acroleins und des Methacroleins.

Das Mengenverhältnis Aldehyd zu Alkohol kann weitgehend beliebig gewählt werden. Im allgemeinen ist es vorteilhaft, den Alkohol in überstöchionmetrischen Mengen einzusetzen.

Die Umsetzung erfolgt in Gegenwart fester saurer Katalysatoren. Als solche dienen vorzugsweise saure, insbesondere stark saure, Ionenaustauscherharze oder stark saure Zeolithe. Das Mengenverhältnis Katalysator zu Aldehyd richtet sich nach der Art des Katalysators. Von den Ionenaustauscherharzen werden zweckmäßigerweise wenigstens 1,0 g, vorzugsweise wenigstens 5,0 g, und von den Zeolithen wenigstens 0,5 g, vorzugsweise wenigstens 1,0 g, je Mol des Aldehyds angewendet.

Für die Umsetzung kommen grundsätzlich Temperaturen etwa bis zum Siedepunkt des Umsetzungsgemischs in Frage. Zweckmäßigerweise wird bei Temperaturen etwa zwischen 0 und 50°C, insbesondere zwischen 10 und 30°C, gearbeitet.

Die bei der Umsetzung der Aldehyde mit den Alkoholen anfallenden Umsetzungsgemische enthalten neben den gebildeten Acetalen die nicht umgesetzten Anteile der Aldehyde und Alkohole. Erfindungsgemäß werden die Umsetzungsgemische nach Abtrennung der Katalysatoren mittels Wasser und mittels inerten mit Wasser nicht mischbaren organischen Lösungsmitteln extrahiert. Hierbei werden die Acetale vom organischen Lösungsmittel und die Aldehyde, insbesondere diejenigen Aldehyde, bei denen $R_1$ gemäß Formel I eine Alkyl- oder Alkenyl-Gruppe mit bis 2 Kohlenstoffatomen ist, sowie die Alkohole bevorzugt vom Wasser aufgenommen.

Welche organischen Lösungsmittel verwendet werden, richtet sich gegebenenfalls nach den Eigenschaften der in den Umsetzungsgemischen enthaltenen Substanzen. Als Lösungsmittel kommen vornehmlich aliphatische oder aromatische, gegebenenfalls halogenierte, Kohlenwasserstoffe in Frage. Bevorzugt werden aliphatische Kohlenwasserstoffe mit 5 bis 14 Kohlenstoffatomen. Geeignete Lösungsmittel sind beispielsweise Benzol, Toluol, Xylol, Methylenchlorid, Trichlormethan, Tetrachlormethan und Chlorbenzol, insbesondere n-Pentan, n-Heptan und n-Octan.

Das Wasser und die organischen Lösungsmittel werden zweckmäßigerweise in solchen Mengen eingesetzt, daß bei der Extraktion die gebildeten Acetale möglichst vollständig vom organischen Lösungsmittel aufgenommen und hierbei möglichst weitgehend von den nicht umgesetzten Anteilen der Aldehyde und Alkohole getrennt werden. Da die Extraktion in kontinuierlicher Arbeitsweise im Gegenstrom erfolgt, ist es zweckmäßig, je Volumenteil des Umsetzungsgemischs 1 bis 4 Volumenteile Wasser und 1 bis 3 Volumenteile organisches Lösungsmittel anzuwenden.

Die mit dem Wasser extrahierten Aldehyde und Alkohole werden zweckmäßigerweise durch Destillation vom Wasser getrennt. Sie können unmittelbar erneut für die Herstellung der Acetale eingesetzt werden.

Durch die organischen Lösungsmittel werden im allgemeinen neben den Acetalen gewisse Anteile der Aldehyde und Alkohole extrahiert. Auch die Gewinnung dieser Substanzen erfolgt zweckmäßigerweise durch Destillation. Hierzu ist es vorteilhaft, organische Lösungsmittel zu wählen, die deutlich höhere Siedepunkte als die Acetale, Aldehyde und Alkohole haben. In den Fällen, in denen die Acetale gleiche Siedepunkte wie die Aldehyde und Alkohole haben und es folglich nicht möglich ist, die Acetale von diesen Substanzen durch Destillation zu trennen, ist es zweckmäßig, bei der Extraktion so viel Wasser anzuwenden, daß die Aldehyde und Alkohole vollständig vom Wasser aufgenommen werden.

Beispiele

1. Es wurden stündlich 169,3 g (3,02 mol) Acrolein mit 189,1 g (5,91 mol) Methanol vermischt in gleichmäßigen Strom einem Schlaufenreaktor zugeführt. Diesem Strom wurden laufend die aus dem Umsetzungsgemisch zurückgewonnenen 117,1 g (2,09 mol) Acrolein und 450,9 g (14,1 mol) Methanol zugemischt. In dem Kreislauf des Reaktors durchströmte die Mischung eine mit 100 g stark saurem Ionenaustauscherharz (Dowex MSC-1) gefüllte Zone. Die Temperatur in dem Reaktor wurde auf 17°C gehalten, die mittlere Verweilzeit war 2,3 Stunden. Das Umsetzungsgemisch wurde in gleichmäßigem Strom aus dem Reaktor abgezogen und in die Mitte einer pulsierenden Extraktionskolonne von 25 cm lichter Weite und 400 cm Höhe mit 80 Siebböden geleitet. In die Kolonne wurden stündlich von oben 1810 g Wasser und von unten 810 g n-Octan eingespeist. Die Temperatur in der Kolonne war 20°C. Die aus der Kolonne oben und unten abgezogenen Phasen wurden fraktionierend destilliert. Hierbei fielen stündlich aus der oberen Phase 290,0 g (2,84 mol) Acrolein-dimethylacetal an. Außerdem wurden stündlich aus der oberen Phase 6,0 g Acrolein und aus der unteren Phase 111,1 g Acrolein und 450,9 g Methanol zurückgewonnen. Es waren folglich 59,1% der insgesamt stündlich eingesetzten 286,4 g Acrolein umgesetzt. Das zurückgewonnene Acrolein und Methanol wurde laufend in den Schlaufenreaktor zurückgeleitet. Bei der Destillation der oberen Phase fiel als Rückstand Octan und bei der Destillation der unteren Phase Wasser an. Das Octan und der überwiegende Teil des Wassers wurden in die Extraktionskolonne zurückgeleitet, das restliche Wasser verworfen. Das gewonnene

Acetal war 98,0%ig. Sein Siedepunkt war 89 bis 90°C.

Die Ausbeute, bezogen auf das umgesetzte Acrolein, betrug 94,0% und mithin war die Selektivität 94,0%.

2. Es wurde wie nach Beispiel 1 verfahren, jedoch wurden stündlich 184 g (4,2 mol) Acetaldehyd und 275 g (8,6 mol) Methanol zugeführt und zusammen mit den aus dem Umsetzungsgemisch zurückgewonnenen Anteilen Acetaldehyd und Methanol eingespeist, die Temperatur im Reaktor wurde auf 18°C gehalten, die mittlere Verweilzeit betrug 3,8 Stunden und das Umsetzungsgemisch wurde in eine Extraktionskolonne geleitet, die stündlich mit 1530 g Wasser und 1510 g n-Octan beschickt wurde. Stündlich wurden aus der organischen Phase 9 g Acetaldehyd, 2 g Methanol und 367 g Acetaldehyd-dimethylacetal und aus der wäßrigen Phase 36 g Methanol und 43 g Acetaldehyd gewonnen. Es waren folglich 76% des insgesamt stündlich eingesetzten Acetaldehyds umgesetzt. Das gewonnene Acetal war 98,5%ig, Sein Siedepunkt war 64 bis 65°C. Die Ausbeute, bezogen auf den umgesetzten Acetaldehyd, betrug 98% und mithin die Selektivität 98%.

**Patentansprüche**

1. Verfahren zur Herstellung von Acetalen durch Umsetzung von Aldehyden der Formel

$$R_1\text{—CHO} \qquad (I),$$

in der $R_1$ für den Methyl- oder Ethylrest steht oder eine Alkenylgruppe mit 2 oder 3 Kohlenstoffatomen bedeutet, mit Alkoholen der Formel

$$R_2\text{—OH} \qquad (II),$$

in der $R_2$ eine Alkylgruppe mit 1 oder 2 Kohlenstoffatomen bedeutet, in Gegenwert fester saurer Katalysatoren, die nach der Umsetzung abgetrennt werden, in flüssiger Phase in Abwesenheit zusätzlicher organischer Lösungsmittel, dadurch gekennzeichnet,

a) daß das von dem Katalysator abgetrennte Umsetzungsgemisch kontinuierlich im Gegenstrom extrahiert wird, wobei man le Volumenteil des Umsetzungsgemisches 1 bis 4 Volumenteile Wasser und 1 bis 3 Volumenteile mit Wasser nicht mischbarer organischer Lösungsmittel einsetzt, und

b) aus den Extrakten die gebildeten Acetale sowie die nicht umgesetzten Anteile der Aldehyde und Alkohole gewinnt.

2. Verfahren nach Anspruch 1, dadurch gekennzeichnet, daß als Katalysatoren stark saure Ionenaustauscherharze oder stark saure Zeolithe verwendet werden.

3. Verfahren nach Anspruch 1 oder 2, dadurch gekennzeichnet, daß als Lösungsmittel aliphatische Kohlenwasserstoffe mit 5 bis 14 Kohlenstoffatomen verwendet werden.

4. Verfahren nach einem oder mehreren der Ansprüche 1 bis 3, dadurch gekennzeichnet, daß Acetaldehyd mit Methanol umgesetzt wird.

5. Verfahren nach einem oder mehreren der Ansprüche 1 bis 3, dadurch gekennzeichnet, daß Acrolein mit Methanol umgesetzt wird.

6. Verfahren nach einem oder mehreren der Ansprüche 1 bis 3, dadurch gekennzeichnet, daß Methacrolein mit Methanol umgesetzt wird.

**Revendications**

1. Procédé d'obtention d'acétals par réaction d'aldéhydes de formule

$$R_1\text{—CHO} \qquad (I)$$

dans laquelle $R_1$ représente un radical méthyle ou éthyle ou signifie un groupe alcényle ayant 2 ou 3 atomes de carbones avec des alcools de formule

$$R_2\text{—CHO} \qquad (II)$$

dans laquelle $R_2$ signifie un radical alcoyle ayant 1 ou 2 atomes de carbone en présence de catalyseurs acides solides, acides qui sont séparés après la réaction, en phase liquide en présence de solvants additionnels, caractérisé en ce que

a) le mélange de la réaction après que le catalyseur ait été séparé, est épuisé en continu à contrecourant, procédé dans lequel on met en jeu par partie en volume du mélange réactionnel 1 à 4 parties en volume d'eau et 1 à 3 parties en volume de solvant organique non miscible à l'eau, et

b) les acétals formés ainsi que les quantités d'aldéhyde et d'alcool qui n'ont pas réagi, sont obtenus à partir des extracteurs.

2. Procédé selon la revendication 1, caractérisé en ce qu'on utilise comme catalyseurs des résines échangeuses fortement acides ou des zéolites fortement acides.

3. Procédé selon les revendications 1 ou 2, caractérisé en ce qu'on utilise comme solution du carbure d'hydrogène aliphatique avec 5 jusqu'à 14 atomes de carbone.

4. Procédé selon l'une ou plusieurs des revendications 1 à 3, caractérisé en ce que l'acétaldéhyde réagit avec le méthanol.

5. Procédé selon l'une ou plusieurs des revendications 1 à 3, caractérisé en ce que l'acroléine réagit avec le méthanol.

6. Procédé selon l'une ou plusieurs des revendications 1 à 3, caractérisé en ce que la méthacroléine réagit avec le méthanol.

**Claims**

1. A process for the production of acetals by reaction of aldehydes corresponding to the following formula:

$$R_1\text{—CHO} \qquad (I)$$

in which $R_1$ represents the methyl or ethyl radical

or a $C_2$ or $C_3$ alkenyl group, with alcohols corresponding to the following formula

$$R_2\text{—}OH \qquad (II)$$

in which $R_2$ is a $C_1$ or $C_2$ alkyl group, in the presence of solid acidic catalysts, which are removed after the reaction, in the liquid phase in the absence of additional organic solvents, characterized in that

a) the reaction mixture separated off from the catalyst is continuously extracted in countercurrent, 1 to 4 parts by volume water and 1 to 3 parts by volume water-immiscible organic solvent being used per part by volume of the reaction mixture, and

b) the acetals formed and the unreacted parts of the aldehydes and alcohols are recovered from the extracts.

2. A process as claimed in claim 1, characterized in that strongly acidic ion exchanger resins or strongly acidic zeolites are used as catalysts.

3. A process as claimed in claim 1 or 2, characterized in that aliphatic hydrocarbons containing 5 to 14 carbon atoms are used as solvents.

4. A process as claimed in one or more of claims 1 to 3, characterized in that acetaldehyde is reacted with methanol.

5. A process as claimed in one or more of claims 1 to 3, characterized in that acrolein is reacted with methanol.

6. A process as claimed in one or more of claims 1 to 3, characterized in that methacrolein is reacted with methanol.